# EUROPEAN PATENT APPLICATION

(11) **EP 4 715 061 A1**
(43) Date of publication of application: **25.03.2026**
(21) Application number: 23945542.1
(22) Date of filing: 16.08.2023
(51) Int. Cl.: C12P 21/02, C07K 5/06, C12N 9/00, C12N 15/70

(54) **PREPARATION METHOD FOR L-AMINO ACID LIGASE AND USE THEREOF IN DIPEPTIDE SYNTHESIS**

(30) Priority: 20.07.2023 CN 202310894436
(71) Applicant: Asymchem Life Science (Tianjin) Co., Ltd, Tianjin 300457 (CN)
(72) Inventor: HONG, Hao, Morrisville North Carolina 27560 (US); JAMES, Gage, Morrisville North Carolina 27560 (US); ZHANG, Na, Tianjin 300457 (CN); JIAO, Xuecheng, Tianjin 300457 (CN); MA, Yulei, Tianjin 300457 (CN); ZHANG, Haibin, Tianjin 300457 (CN); QI, Yike, Tianjin 300457 (CN); MA, Tianjiao, Tianjin 300457 (CN)
(74) Representative: Biggi, Cristina
(86) International application number: PCT/CN2023/113398
(87) International publication number: WO 2025/015657

(57) **Abstract**

Provided are a method for preparing an L-amino acid ligase and a use thereof in dipeptide synthesis. The method includes using an L-amino acid ligase with the amino acid sequence as shown in SEQ ID NO: 1 for a dipeptide synthesis reaction, and obtaining the dipeptide. The L-amino acid ligase of the present application can be used for synthesis of a plurality of functional dipeptides, has a broad substrate spectrum and high synthesis efficiency, can be well used for industrial amplification, has low cost and high yield, and realizes true green chemistry.

## Description

The present application is based on the Chinese application with the application number of CN 202310894436.9 filed on July 20, 2023 and claims the priority thereto, the disclosure of which is incorporated herein by reference in its entirety.

### Technical Field

The present invention relates to the technical field of enzymes, in particular to a method for preparing an L-amino acid ligase and use thereof in dipeptide synthesis.

### Background

A dipeptide is the simplest peptide, which is composed of an amide bond formed by dehydration condensation between the α-carboxyl group of one amino acid molecule and the α-amino group of another amino acid molecule. Although the dipeptide has a simple structure, it has a wide-ranging biological activity, and can exert a regulatory effect on the vital activities of organisms, such as physiological metabolism. The demand for peptides, which are based on antibiotics, antivirals, anticancer agents, hormones and immunomodulatory effects, has been increasing year by year, and they are widely applied to the fields of medicine, food, health products, cosmetics and the like. For example, carnosine (β-alanyl-His) has antioxidant, anti-inflammatory and anti-glycation effects; glycylglycine (Gly-Gly) is used in medicine as a stabilizer for blood preservation and a protein drug cytochrome C aqueous injection; alanyl-glutamine (Ala-Gln) serves as an important nutritional supplement for surgical patients; aspartame (Asp-Phe methyl ester) is a widely used sweetener; Ala-Phe, Ile-Phe and Pro-Gly dipeptides are saltiness enhancers; Ile-Tyr, Lys-Trp, Val-Tyr and Ile-Trp are antihypertensive peptides; Arg-Trp has analgesic effects; and Lys-Glu has antitumor activity, among others.

Currently, dipeptide synthesis is mainly chemical synthesis. This process involves protection and deprotection related operation processes. Its product may undergo racemization and has relatively high synthesis cost, and even this process requires toxic reagents, among others. A biosynthesis method has excellent stereoselectivity, without the generation of racemic products. The L-amino acid ligase can ligate two free amino acids in the presence of ATP to synthesize the dipeptide. The existing L-amino acid ligases (Lal) have a narrow substrate spectrum. Furthermore, the concentration of the substrate in the dipeptide synthesis reaction is low, and the activity of the enzyme is insufficient after the substrate concentration increases, which severely restricts the widespread application of Lal in synthesis of a plurality of functional dipeptides. Therefore, the development of an L-amino acid ligase with a broad substrate spectrum is of great significance for dipeptide synthesis.

### Summary

The main objective of the present invention is to provide a use of an L-amino acid ligase in dipeptide synthesis and a method for preparing the L-amino acid ligase, so as to solve the problem in the prior art that the L-amino acid ligase has a narrow substrate spectrum.

To realize the above objective, according to a first aspect of the present invention, provided is a method for preparing a dipeptide, comprising performing a dipeptide synthesis reaction using an L-amino acid ligase with an amino acid sequence as shown in SEQ ID NO: 1 to obtain the dipeptide.

Further, a substrate for the dipeptide synthesis reaction is an amino acid; preferably, the amino acid is selected from one or two of glycine, alanine, valine, leucine, isoleucine, serine, threonine, cysteine, methionine, asparagine, glutamine, aspartic acid, glutamic acid, lysine, arginine, histidine, phenylalanine, tryptophan, proline or tyrosine.

Further, the dipeptide comprises Gly-Gln, Gly-Tyr, Gly-Gly or Ala-Gln.

Further, a reaction system for the dipeptide synthesis reaction comprises a buffer with the pH of 8.5-9.0 as follows: 0.1-0.2 M Tris-HCl, 0.1-0.2 M ATP and 0.1-0.2 M MgCl₂.

Further, a reaction temperature for the dipeptide synthesis reaction is 20-25°C.

Further, a reaction time for the dipeptide synthesis reaction is 15-17 h.

Further, a mass concentration of the L-amino acid ligase for the dipeptide synthesis reaction in the reaction system is 0.05-1 mg/mL.

Further, a concentration of the substrate for the dipeptide synthesis reaction in the reaction system is 10-200 mM.

To realize the objective hereinabove, according to a second aspect of the present invention, provided is a method for preparing an L-amino acid ligase, comprising cloning a gene encoding the L-amino acid ligase with an amino acid sequence as shown in SEQ ID NO: 1 into an expression vector to obtain a recombinant vector; introducing the recombinant vector into *Escherichia coli* to obtain a recombinant strain; incubating the recombinant strain to induce the expression of the L-amino acid ligase, thereby obtaining a culture solution; and performing ultrasonication on the culture solution, followed by centrifugation, so as to obtain the L-amino acid ligase.

Further, the expression vector comprises pET-22a(+), pET-22b(+), pET-3a(+), pET-3d(+), pET-11a(+), pET-12a(+), pET-14b(+), pET-15b(+), pET-16b(+), pET-17b(+), pET-19b(+), pET-20b(+), pET-21a(+), pET-23a(+), pET-23b(+), pET-24a(+), pET-25b(+), pET-26b(+), pET-27b(+), pET-28a(+), pET-29a(+), pET-30a(+), pET-31b(+), pET-32a(+), pET-35b(+), pET-38b(+), pET-39b(+), pET-40b(+), pET-41a(+), pET-41b(+), pET-42a(+), pET-43a(+), pET-43b(+), pET-44a(+), pET-49b(+), pQE2, pQE9, pQE30, pQE31, pQE32, pQE40, pQE70, pQE80, pRSET-A, pRSET-B, pRSET-C, pGEX-5X-1, pGEX-6p-1, pGEX-6p-2, pBV220, pBV221, pBV222, pTrc99A, pTwin1, pEZZ18, pKK232-18, pUC-18 or pUC-19; preferably, a host cell comprises a prokaryotic cell or an eukaryotic cell; preferably, the prokaryotic cell is *Escherichia coli and Bacillus subtilis*; more preferably, the strain of the *Escherichia coli* is BL21; preferably, the eukaryotic cell is *Saccharomyces cerevisiae* and *Pichia pastoris.*

By applying the technical solution of the present invention, the synthesis of a plurality of functional dipeptides can be performed using the L-amino acid ligase of the present application. The L-amino acid ligase of the present application has a broad substrate spectrum and high synthesis efficiency, can be well used for industrial amplification, and is low in cost and high in yield, thereby realizing true green chemistry.

### Brief Description of the Drawings

The drawings of the specification constituting a part of the present application serve to provide further understanding of the present invention. Exemplary embodiments of the present invention and descriptions thereof are used for explaining the present invention, and do not constitute improper limitation to the present invention. In the drawings:
FIG. 1 is a diagram showing sodium dodecyl sulfate-polyacrylamide gel electrophoresis (SDS-PAGE) results of expression of a recombinant L-amino acid ligase in *Escherichia coli* in Example 1; and
FIG. 2 is a diagram showing SDS-PAGE results of purifying Blal proteins from *Escherichia coli* in Example 2.

### Detailed Description of the Embodiments

It should be noted that the embodiments of the present application and the features of the embodiments can be combined with each other without conflict. The present invention will be described in detail below with reference to embodiments.

As mentioned in the background art, L-amino acid ligases in the prior art generally have a narrow substrate spectrum in dipeptide synthesis reactions. Different L-amino acid ligases exhibit different substrate specificities. Some of them are unable to utilize acidic or alkaline amino acids as substrates; some of them are able to synthesize dipeptides with larger amino acids at the N terminal and smaller amino acids at the C terminal, and others exhibit the opposite pattern; and some of them show highly restricted substrate specificities, for example, only L-methionine and L-leucine are received at the N-terminal and the substrates at the C terminal are only limited to small residues.

In addition, the L-amino acid ligase in the prior art can only catalyze reactions at a low substrate concentration (typically only several tens of mM) and has insufficient activity, and cannot be used for synthesis of a plurality of functional dipeptides, which restricts its wide application in producing various dipeptide products. Therefore, it is of great significance to develop an L-amino acid ligase with a broad substrate spectrum. In the present application, the inventors have modified the L-amino acid ligase through screening and codon optimization methods, thereby enhancing various enzyme properties, broadening the substrate spectrum of the enzyme, enhancing the enzymatic activity of the L-amino acid ligase, enabling the L-amino acid ligase to be widely applied to technical solutions for producing functional dipeptides. Thus, a series of protection schemes of the present application are proposed.

In a first typical embodiment of the present invention, provided is a method for preparing a dipeptide, comprising performing a dipeptide synthesis reaction using an L-amino acid ligase with an amino acid sequence as shown in SEQ ID NO: 1 to obtain the dipeptide.

The amino acid sequence as shown in SEQ ID NO: 1 hereinabove is an amino acid sequence obtained by transcription and translation of a codon-optimized DNA sequence of an L-amino acid ligase Blal derived from *Bacillus sp.* Root920. The codon optimization was performed based on the codon usage preference of *Escherichia coli.*

Codon optimization is performed on the DNA sequence of the L-amino acid ligase Blal derived from *Bacillus sp.* Root920 according to the preference of *Escherichia coli.*

Families of amino acid residues having similar side chains have been defined in the prior art. These families comprise amino acids having basic side chains (e.g., lysine, arginine and histidine), acidic side chains (e.g., aspartic acid and glutamic acid), uncharged polar side chains (e.g., glycine, asparagine, glutamine, serine, threonine, tyrosine, cysteine and tryptophan), nonpolar side chains (e.g., alanine, valine, leucine, isoleucine, proline, phenylalanine and methionine), β-branched side chains (e.g., threonine, valine and isoleucine) and aromatic side chains (e.g., tyrosine, phenylalanine, tryptophan and histidine). Therefore, it is preferable to replace a corresponding amino acid residue with another amino acid residue derived from the same side chain family. Methods for identifying conservative substitutions of amino acids are well known in the prior art (see, e.g., Brummell et al., Biochem. 32:1180-1187 (1993); Kobayashi et al., Protein Eng. 12(10):879-884 (1999); and Burks et al., Proc. Natl. Acad. Set USA 94:412-417 (1997)).
SEQ ID NO: 1: 
MSKKTVLVIADLGGCPPHMFYESVAASYHIVSYIPRPFAITKGHAELIEKYSIAVIKDRDYFETHP
SFEHPDSIYWAHDDYLKSEEEVVDDLVRVASFFKADAITTNNELFIAPMAKAAERLGLRGAGVKAA
EKARDKSQMRAAFNAAGVKAVKTQPVTTLADFQQAIEHIGTPLILKPTYLASSIGVTLFHDRAGSDD
LFLNVQSYLQTIPVPNAVTYEAPFVAETYLEGAYKDWYQEDGYSDYVSVEGLVVEGEYIPFVIHDKT
PQIGFTETAHITPSILDNEAQQIIIEAAKKANEGLGLENCATHTEIKLMKNRETGLIESAARFAGWNMI
PNIKKVFGVDMAKLLIDVLVDGKKAELPKELLSGHTHYIADCHLYPQHFKESGHIPAEATHITIDHIHIP
QDALVGDTVIVSKSVPSKGTFVDLSLFEAFNGIVSLELKGSSSQDVAVSIRNLQKQAAIHLMDELVK
G.
SEQ ID NO: 2: (the codon-optimized DNA sequence of the L-amino acid ligase Blal derived from *Bacillus sp.* Root920)

ATGTCAAAAAAGACAGTTCTAGTAATAGCTGATTTGGGTGGCTGCCCACCGCACATGTTTT
ATGAAAGCGTTGCGGCATCCTACCATATTGTGAGCTACATACCGCGTCCGTTCGCAATCACTAA
AGGCCACGCAGAGCTGATTGAAAAATACAGCATCGCGGTCATCAAGGACCGTGACTACTTCGA
AACCCACCCGTCTTTTGAACATCCGGATAGCATTTACTGGGCACACGATGACTACCTGAAGAG
CGAAGAAGAGGTGGTGGACGACCTGGTGCGCGTCGCCTCTTTTTTCAAAGCGGACGCAATCA
CCACCAATAACGAGCTGTTCATCGCCCCGATGGCGAAAGCGGCGGAACGTCTGGGCCTGCGT
GGTGCGGGCGTGAAGGCTGCGGAAAAGGCTCGCGATAAGTCCCAAATGAGAGCTGCGTTTAA
CGCCGCAGGTGTTAAAGCTGTGAAGACCCAGCCGGTTACCACTCTGGCGGATTTCCAGCAAG
CAATCGAGCACATCGGTACGCCGCTGATCCTGAAGCCGACCTACTTGGCTTCCTCTATCGGCG
TGACCCTGTTTCATGACCGCGCCGGTTCCGACGATTTGTTCCTGAATGTTCAGAGCTACCTGC
AAACCATTCCGGTGCCGAACGCGGTTACGTACGAGGCGCCTTTCGTGGCTGAAACGTACCTT
GAGGGCGCTTATAAAGACTGGTATCAAGAAGATGGTTATAGCGATTATGTTAGCGTCGAGGGTC
TCGTAGTTGAGGGCGAATATATTCCGTTCGTCATTCACGATAAAACCCCGCAGATCGGCTTTAC
TGAAACCGCGCATATTACCCCGAGCATCCTGGATAATGAGGCGCAGCAAATTATTATCGAAGCG
GCGAAAAAGGCGAACGAGGGTCTGGGTTTGGAGAACTGCGCTACCCATACCGAAATCAAGCT
GATGAAAAACCGTGAAACCGGTTTGATCGAGAGCGCAGCGCGTTTTGCAGGCTGGAATATGAT
TCCCAACATCAAGAAAGTTTTCGGTGTTGACATGGCCAAGCTGCTGATTGACGTGCTGGTTGA
CGGGAAAAAGGCGGAGCTTCCAAAAGAATTACTGAGCGGTCACACCCACTATATTGCGGACTG

TCATCTATATCCGCAACATTTTAAAGAGTCTGGTCACATCCCTGCAGAGGCCACCCACATCACG
ATTGATCATATTCACATCCCGCAGGACGCGCTGGTGGGTGATACCGTTATCGTGTCGAAGAGC
GTACCGAGCAAAGGCACGTTTGTTGACCTGAGTCTTTTCGAGGCCTTCAACGGTATTGTGAGC
TTAGAGTTGAAGGGCTCATCGTCCCAGGACGTTGCTGTTTCGATCCGTAATCTGCAGAAACAG
GCAGCCATCCACCTGATGGATGAATTGGTGAAGGGC.

The L-amino acid ligase of the present application can utilize a plurality of amino acids as substrates for dipeptide synthesis. Therefore, any amino acid that can be acted upon by the L-amino acid ligase is applicable to the present application. In a preferred embodiment, the substrate for the dipeptide synthesis reaction is an amino acid. In a preferred embodiment, the amino acid is selected from one or two of glycine, alanine, valine, leucine, isoleucine, serine, threonine, cysteine, methionine, asparagine, glutamine, aspartic acid, glutamic acid, lysine, arginine, histidine, phenylalanine, tryptophan, proline or tyrosine. The L-amino acid ligase of the present application can use 20 amino acids hereinabove as substrates, and still can maintain a certain activity for dipeptide synthesis after the substrate concentration increases.

Any dipeptide synthesized by using the L-amino acid ligase is applicable to the present application. In a preferred embodiment, the dipeptide comprises Gly-Gln, Gly-Tyr, Gly-Gly or Ala-Gln. Particularly, some important functional dipeptides are synthesized by using the L-amino acid ligase. Where, the dipeptide is composed of an amide bond formed by dehydration and condensation between the α-carboxyl group of one amino acid molecule and the α-amino group of another amino acid molecule, and is widely applied to the fields of medicine, food, health products and cosmetics. For example, glycyl-glutamine (Gly-Gln) can replace glutamine as a nutrient for cell culture, increasing the yield of cells; glycyl-tyrosine (Gly-Tyr) is an antihypertensive peptide; glycyl-glycine (Gly-Gly) is used in medicine as a stabilizer for blood preservation and protein drug cytochrome C aqueous injections; and alanyl-glutamine (Ala-Gln) serves as an important nutritional supplement for surgical patients.

The dipeptide synthesis reaction of the present application is that two free amino acids are ligated by consuming ATP under the alkaline condition to form the dipeptide. Any reaction system capable of performing the dipeptide synthesis reaction using the L-amino acid ligase is applicable to the present application. In a preferred embodiment, the reaction system for the dipeptide synthesis reaction comprises: 0.1-0.2 M Tris-HCl, 0.1-0.2 M ATP, and 0.1-0.2 M MgCl₂; and the pH of the Tris-HCl is 8.5-9.0.

Any reaction conditions capable of completing the dipeptide synthesis reaction are applicable to the present application. From the perspective of the reaction efficiency and energy consumption of the dipeptide, in a preferred embodiment, a reaction temperature of the dipeptide synthesis reaction is 20-25°C; and a reaction time of the dipeptide synthesis reaction is 15-17 h.

The quality of any enzyme utilizing the L-amino acid ligase to perform the dipeptide synthesis reaction and the concentration of the substrate are applicable to the present application. From the perspective of improving the reaction efficiency of dipeptide synthesis, in a preferred embodiment, a mass concentration of the L-amino acid ligase for the dipeptide synthesis reaction in the reaction system is 0.05-1 mg/mL; and a concentration of the substrate for the dipeptide synthesis reaction in the reaction system is 10-200 mM.

In a second typical embodiment of the present invention, provided is a method for preparing an L-amino acid ligase, comprising cloning a gene of the L-amino acid ligase hereinabove into an expression vector to obtain a recombinant vector; introducing the recombinant vector into *Escherichia coli* to obtain a recombinant strain; incubating the recombinant strain and inducing the expression of the L-amino acid ligase to obtain a culture solution; and performing ultrasonication on the culture solution, followed by centrifugation, to obtain the L-amino acid ligase. The expression and production of the L-amino acid ligase are accomplished by using a conventional prokaryotic protein expression method.

Unless otherwise specified, molecular biology experimental methods used in the present application are substantially performed with reference to methods described in J. Sambrook et al., Molecular Cloning: A Laboratory Manual, 2nd Edition, Cold Spring Harbor Laboratory Press, 1989, and F. M. Ausubel et al., Short Protocols in Molecular Biology, 3rd Edition, John Wiley & Sons, Inc., 1995; and the use of restriction endonucleases follows the conditions recommended by product manufacturers. A person skilled in the art understands that in the embodiments, the embodiments describe the present application by way of examples and are not intended to limit the scope claimed by the present application.

Any recombinant vector capable of normally expressing the L-amino acid ligase is applicable to the present application. In a preferred embodiment, the expression vector comprises pET-22a(+), pET-22b(+), pET-3a(+), pET-3d(+), pET-11a(+), pET-12a(+), pET-14b(+), pET-15b(+), pET-16b(+), pET-17b(+), pET-19b(+), pET-20b(+), pET-21a(+), pET-23a(+), pET-23b(+), pET-24a(+), pET-25b(+), pET-26b(+), pET-27b(+), pET-28a(+), pET-29a(+), pET-30a(+), pET-31b(+), pET-32a(+), pET-35b(+), pET-38b(+), pET-39b(+), pET-40b(+), pET-41a(+), pET-41b(+), pET-42a(+), pET-43a(+), pET-43b(+), pET-44a(+), pET-49b(+), pQE2, pQE9, pQE30, pQE31, pQE32, pQE40, pQE70, pQE80, pRSET-A, pRSET-B, pRSET-C, pGEX-5X-1, pGEX-6p-1, pGEX-6p-2, pBV220, pBV221, pBV222, pTrc99A, pTwin1, pEZZ18, pKK232-18, pUC-18 or pUC-19.

Any host cell capable of normally inducing the expression of the L-amino acid ligase is applicable to the present application. In a preferred embodiment, a host cell comprises a prokaryotic cell or a eukaryotic cell; the prokaryotic cell is *Escherichia coli* and *Bacillus subtilis;* more preferably, the strain of *Escherichia coli* is BL21; and the eukaryotic cell is *Saccharomyces cerevisiae* and *Pichia pastoris.* pET-28a (+). The *Escherichia coli* strain comprises BL21.

Next, the present application will be further described in detail in conjunction with specific embodiments. These should not be construed as limiting the scope of protection claimed by the present application.

### Example 1 Screening and expression of genes encoding L-amino acid ligase Blal

The sequences of reported enzymes with L-amino acid ligase activity were used as probes, homologous searching was performed in a database to preliminarily obtain 10 enzymes belonging to an ATP-grasp superfamily (ATP-dependent carboxylate-amine ligases). The obtained 10 enzymes were determined to be good candidates for synthesizing short oligopeptides. Further sequence analysis was performed on the 10 enzymes, and the L-amino acid ligase Blal derived from *Bacillus sp.* Root920 was selected for subsequent expression and functional studies.

Codon optimization was performed on the DNA sequence of the L-amino acid ligase Blal derived from *Bacillus sp.* Root920. The optimized gene fragment was synthesized by a gene synthesis company, i.e., an endonuclease site Ndel was introduced at the 5' terminal and an endonuclease site Xhol was introduced at the 3' terminal, and the gene was synthesized into pUC19 to obtain pUC19-Blal. The expression vector pET-28a (+) was subjected to double digestion (Ndel+Xhol), and meanwhile the gene pUC19-Blal of the L-amino acid ligase was subjected to double digestion (Ndel+Xhol). The gene fragment encoding Blal was cleaved and ligated with the expression vector pET-28a(+) to obtain an *Escherichia coli* recombinant plasmid pET-28a(+)-Blal containing L-amino acid ligase Blal, and then the *Escherichia coli* recombinant plasmid pET-28a(+)-Blal containing the L-amino acid ligase Blal was transformed into *Escherichia coli* BL21(DE3) to obtain a recombinant *Escherichia coli* strain BL21(DE3)/Blal.

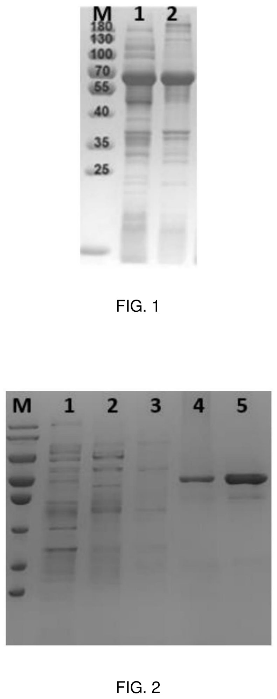

4 mL of BL21(DE3) strain containing the recombinant plasmid was taken and inoculated into a 2L Erlenmeyer flask containing 400 mL of LB culture medium, and then underwent shaking culture at 37°C and 200 rpm for 2-3 h. When the OD600 reached 0.6-0.8, IPTG was added to a final concentration of 0.2 mM and then induce the strain at 25°C for 20 h. After induction was ended, the product after induction was centrifuged at 4°C to collect bacterial cells. Through ultrasonication and centrifugation, the supernatant and precipitates were collected. The 12% separation gel SDS-PAGE results demonstrated that the recombinant L-amino acid ligase was expressed in *Escherichia coli,* as shown in FIG. 1. In FIG. 1, M is a protein marker, lane 1 is a supernatant of a cell lysate of an *Escherichia coli* strain expressing an L-amino acid ligase, and lane 2 is a precipitate of the cell lysate of the *Escherichia coli* strain expressing the L-amino acid ligase. The enzyme gene encodes a protein that consists of 473 amino acids and has a theoretical molecular weight of 52 kDa. Note: The test materials and reagents used in the example are described as follows:
1. Strain and vector: *Escherichia coli* expression vector pET-28a (+) and strain BL21 (DE3)
2. Culture medium: *Escherichia coli* culture medium LB (10 g/L of peptone, 5 g/L of yeast extract, 10 g/L of NaCl, pH 7.0).

### Example 2 Purification of Blal protein expressed in Escherichia coli

The expressed Blal bacterial sludge was resuspended at a bacterial concentration of 10%. The resuspended *Blal* bacterial sludge underwent ultrasonication (2 s ultrasound, 6 s interval, 25% power), centrifuged at 12000 rpm for 30 min with twice repetitions of centrifugation, and then filtered through a 0.45 µm membrane.

Purification was performed using affinity chromatography. The specific process was as follows: a sample was loaded onto the column at a flow rate of 2 ml/min. The column was then washed with buffer A (20 mM Tris-HCl, 200 mM NaCl, pH 8.0) until the unbound proteins were completely eluted. This was followed by a linear gradient elution over 5 column volumes to wash off weakly bound contaminants, with the imidazole concentration increasing from 0 mM to 30 mM. Finally, the target protein was eluted with a buffer containing 200 mM imidazole. The affinity-purified protein was concentrated and the buffer was exchanged using an ultrafiltration device to remove imidazole and salts. Glycerol was added to a final concentration of 20% (v/v) for cryoprotection, and then the protein solution was stored at -20°C for future use. The protein concentration was determined to be 80 mg/mL using the Bradford method. The samples were analyzed by SDS-PAGE on a 12% separating gel. As shown in FIG. 2. lane M contains the protein molecular weight marker; lanes 1-3 how the unbound protein fractions, lane 4 shows proteins eluted with 30 mM imidazole, and lane 5 shows the target protein eluted with 200 mM imidazole.

### Example 3 Determination of substrate spectrum of L-amino acid ligase Blal

The activity of Blal for synthesis of different dipeptides was investigated. The reaction system was as follows (0.5 mL): 10 mM of two identical or different amino acids were used as substrates, 15 mM ATP, 10 mM MgCl₂, 50 µg/mL Blal pure enzyme, 0.1 M Tris-HCl (pH 9.0), and react at 25°C for 16 h. After the reaction was completed, 0.5 mL of methanol was added, and liquid chromatography-mass spectrometry (LC-MS) analysis was performed after centrifugation. The reaction results are summarized as follows (Table 1):

**Table 1**

| | Gly | Ala | Val | Leu | Ile | Ser | Thr | Cys | Met | Asn | Gln | Asp | Glu | Lys | Arg | His | Phe | Trp | Pro | Tyr |
|---|---|---|---|---|---|---|---|---|---|---|---|---|---|---|---|---|---|---|---|---|
| Tyr | *** | * | * | ** | * | *** | * | ** | *** | * | *** | *** | *** | *** | *** | * | ** | *** | * | *** |
| Pro | * | | * | * | ** | *** | * | *** | * | * | * | * | * | *** | *** | * | *** | * | * | |
| Trp | * | * | ** | * | ** | * | ** | * | * | ** | * | ** | ** | *** | * | ** | ** | * | | |
| Phe | * | | * | ** | * | * | * | * | * | ** | * | * | * | ** | ** | *** | *** | | | |
| His | * | ** | * | * | * | * | * | ** | * | * | ** | *** | * | *** | * ** | * | | | | |
| Arg | ** | ** | * | *** | ** | ** | * | * | ** | ** | * | * | ** | * | | | | | | |
| Lys | | ** | * | *** | * | *** | ** | ** | * | *** | * | * | ** | * | | | | | | |
| Glu | * | *** | ** | * | * | *** | * | *** | * | *** | * | * | *** | | | | | | | |
| Asp | *** | * | * | ** | ** | * | * | ** | * | *** | ** | ** | | | | | | | | |
| Gln | *** | *** | * | *** | ** | * | * | ** | * | *** | * | | | | | | | | | |
| Asn | *** | ** | * | * | ** | ** | * | *** | ** | * | | | | | | | | | | |
| Met | *** | * | ** | * | * | * | * | *** | * | | | | | | | | | | | |
| Cys | * | * | * | * | ** | * | * | ** | | | | | | | | | | | | |
| Thr | ** | ** | ** | * | ** | ** | * | | | | | | | | | | | | | |
| Ser | * | ** | * | ** | * | *** | | | | | | | | | | | | | | |
| Ile | ** | * | ** | *** | * | | | | | | | | | | | | | | | |
| Leu | ** | * | * | ** | | | | | | | | | | | | | | | | |
| Val | * | * | ** | | | | | | | | | | | | | | | | | |
| Ala | * | *** | | | | | | | | | | | | | | | | | | |
| Gly | *** | | | | | | | | | | | | | | | | | | | |

**Note:** This table shows the reaction results of Blal. In this table, the first row and the first column are substrate amino acids shown. * represents 0 ≤ molar conversion rate <1%, ** represents that the molar conversion rate is larger than or equal to 1% and less than 10%, and *** represents that the molar conversion rate is larger than 10%. Where, the molar conversion rate is a molar concentration of a dipeptide in a system after a reaction/ (a molar concentration of a single amino acid + the molar concentration of a dipeptide) × 100%. As can be seen from Table 1, the L-amino acid ligase Blal of the present invention has a broad substrate spectrum and can be used for synthesizing homo- and hetero-functional dipeptides.

### Example 4 L-amino acid ligase Blal for Gly-Tyr synthesis

The reaction for Blal to synthesize high-concentration Gly-Tyr was investigated. The reaction system was as follows (100 mL): each 200 mM for glycine and tyrosine, 200 mM ATP, 200 mM MgCl₂, 1 mg/mL Blal pure enzyme, 0.1 M Tris-HCl (pH 9.0), and react at 25°C for 16 h. After the reaction was completed, 100 mL of methanol was added, and high performance liquid chromatography (HPLC) analysis was performed after centrifugation. An external standard curve was generated by measuring ultraviolet absorption peak areas of Gly-Tyr standard samples at different concentrations. The concentration of the product Gly-Tyr was calculated using the external standard curve. It was finally determined that the concentration of Gly-Tyr was 130 Mm after the reaction, the molar conversion rate was 65%, and the yield was approximately 31 g/L.

### Example 5 L-amino acid ligase Blal for Gly-Gly synthesis

The reaction for Blal to synthesize high-concentration Gly-Gly was investigated. The reaction system was as follows (100 mL): 400 mM glycine, 200 mM ATP, 200 mM MgCl₂, 1 mg/mL purified Blal enzyme, 0.1 M Tris-HCl (pH 9.0), and react at 25°C for 16 h. After the reaction was completed, 100 mL of methanol was added, and HPLC analysis was performed after centrifugation. An external standard curve was generated by measuring the ultraviolet absorption peak areas of Gly-Gly standard samples at different concentrations. The concentration of the product Gly-Gly was calculated using the external standard curve. It was finally determined that the concentration of Gly-Gly was 140 mM after the reaction, the molar conversion rate was 70%, and the yield was approximately 18.5 g/L.

### Example 6 L-amino acid ligase Blal for Gly-Gln synthesis

The reaction for synthesizing high-concentration Gly-Gln by Blal was investigated. The reaction system was as follows (100 mL): each 200 mM glycine and glutamine, 200 mM ATP, 200 mM MgCl2, 1 mg/mL Blal pure enzyme, 0.1 M Tris-HCl (pH 9.0), and react at 25°C for 16 h. After the reaction was completed, 100 mL of methanol was added, and HPLC analysis was performed after centrifugation. An external standard curve was generated by measuring the UV absorption peak areas of Gly-Gln standard samples at different concentrations. The concentration of the product Gly-Gln was calculated using the external standard curve. It was finally determined that the concentration of Gly-Gln was 120 mM after the reaction, the molar conversion rate was 60%, and the yield was approximately 24.4 g/L.

### Example 7 L-amino acid ligase Blal for Ala-Gin synthesis

The reaction for synthesizing high-concentration Ala-Gln by Blal was investigated. The reaction system was as follows (100 mL): each 200 mM for alanine and glutamine, 200 mM ATP, 200 mM MgCl₂, 1 mg/mL purified Blal enzyme, 0.1 M Tris-HCl (pH 9.0), and react at 25°C for 16 h. After the reaction was completed, 100 mL of methanol was added, and HPLC analysis was performed after centrifugation. An external standard curve was generated by measuring the ultraviolet absorption peak areas of Ala-Gln standard samples at different concentrations. The concentration of the product Ala-Gln was calculated using the external standard curve. It was finally determined that the concentration of Ala-Gln was 180 mM after the reaction, the molar conversion rate was 90%, and the yield was approximately 39.1 g/L.

### Comparative example 1 Comparison between Blal and existing L-amino acid ligases

Comparing Blal in the present invention with L-amino acid ligases reported in the existing literature, it can be seen that: (1) the activity of Blal in the present invention is significantly higher than the activities of currently reported enzymes at the same substrate concentration, and the activity is still maintained at a relatively high level even under high substrate concentrations; (2) the enzyme in the present invention has a broader substrate range and is able to synthesize a greater number of dipeptides; and (3) the enzyme in the present invention has achieved gram-scale preparation of several dipeptides, demonstrating that the enzyme in the present invention has greater potential and value for industrial application compared to enzymes reported in the literature.

**Table 2:**

| Source | Substrate concentration/activity* | Available substrate range | The number of synthesizable dipeptides | Whether to achieve gram-scale preparation of dipeptides |
|---|---|---|---|---|
| *Bacillus subtilis* 168 | 30 mM/69% | The product prefers small-sized amino acids at the N-terminal and large-sized amino acids at the C-terminal; and it cannot use charged amino acids as substrates. | Confirm to synthesize 44 dipeptides | No |
| *Ralstonia solanacearum* | 12.5 mM/72% | The product prefers small-volume amino acids at the C-terminal and large-volume amino acids at the N-terminal. | Confirm to synthesize 96 dipeptides | No |
| *Bacillus licheniformis* | N/A | The product only receives L-methionine | No more than 40 dipeptides | No |
| NBRC 12200 | | at the N-terminal and L-leucine and only receives small substrates at the C-terminal. | | |
| *Bacillus subtilis* NB RC3134 | N/A | The product only receives L-arginine at the N-terminal | No more than 20 dipeptides | No |
| *Pseudomonas syringae pv. phaseolicola* 1448A | 12.5 mM/4.8% | The product receives serine, threonine and glycine, rather than methionine, at the N-terminal. | Only partially heterodimeric dipeptides are synthesized | No |
| *P. luminescens subsp. laumondii* TT0 | 12.5 mM/62% | Preferentially use L-asparagine as a substrate to synthesize homodipeptides | No more than 20 dipeptides | No |
| The present invention | 30 mM/98% 200 mM/90% | The substrate spectrum is broad without strict substrate limitation. | Confirm that the number of synthesizable dipeptides is greater than 150 | Yes |

| | | | | |
|---|---|---|---|---|
| *The activity data in the table hereinabove are expressed as a molar conversion rate of a product to a substrate. *P. luminescens subsp. laumondii* TT0 uses Asn and Ala as reaction substrates, and the reaction substrates for other cases are Ala and Gln. | | | | |

From the above description, it can be seen that the examples hereinabove of the present invention achieve the following technical effects: (1) the L-amino acid ligase Blal in the present invention has a broad substrate spectrum and can be used for synthesizing homo- and hetero-functional dipeptides; and

(2) the L-amino acid ligase Blal in the present invention has high activity, and therefore still has relatively high molar conversion rate in a 200 mM substrate reaction to synthesize high concentrations of Gly-Tyr, Gly-Gln, Gly-Gly and Ala-Gln.

The descriptions hereinabove are merely preferred embodiments of the present invention and are not intended to limit the present invention. Those skilled in the art can make various modifications and variations to the present invention. Any modifications, equivalent replacements, improvements and the like made within the spirit and principle of the present invention shall be still included in the protection scope of the present invention.

## Claims

1. A method for preparing a dipeptide, wherein the method comprises using an L-amino acid ligase with the amino acid sequence as shown in SEQ ID NO: 1 to perform a dipeptide synthesis reaction, and obtaining the dipeptide.

2. The method according to claim 1, wherein a substrate for the dipeptide synthesis reaction is an amino acid.

3. The method according to claim 2, wherein the amino acid is selected from one or two of glycine, alanine, valine, leucine, isoleucine, serine, threonine, cysteine, methionine, asparagine, glutamine, aspartate, glutamic acid, lysine, arginine, histidine, phenylalanine, tryptophan, proline, or tyrosine.

4. The method according to claim 1, wherein the dipeptide comprises Gly-Gln, Gly-Tyr, Gly-Gly or Ala-Gln.

5. The method according to claim 1, wherein a reaction system for the dipeptide synthesis reaction comprises a following buffer solution with a pH value of 8.5-9.0: 0.1-0.2 M of Tris-HCl, 0.1-0.2 M of ATP, and 0.1-0.2 M of MgCl₂.

6. The method according to claim 1, wherein a reaction temperature for the dipeptide synthesis reaction is 20-25°C.

7. The method according to claim 1, wherein a reaction time for the dipeptide synthesis reaction is 15-17 h.

8. The method according to claim 1, wherein a mass concentration of the L-amino acid ligase for the dipeptide synthesis reaction in the reaction system is 0.05-1 mg/mL.

9. The method according to claim 1, wherein a concentration of the substrate for the dipeptide synthesis reaction in the reaction system is 10-200 mM.

10. A method for preparing an L-amino acid ligase, comprising:
cloning a gene encoding the L-amino acid ligase with the amino acid sequence as shown in SEQ ID NO: 1 into an expression vector, and obtaining a recombinant vector;
introducing the recombinant vector into a host cell, and obtaining a recombinant strain;
cultivating the recombinant strain and inducing the expression of the L-amino acid ligase, and obtaining a culture solution;
sonicating and then centrifuging the culture solution, and obtaining the L-amino acid ligase.
